# EUROPEAN PATENT APPLICATION

(11) **EP 4 329 282 A1**
(43) Date of publication of application: **28.02.2024**
(21) Application number: 22827359.5
(22) Date of filing: 02.06.2022
(51) Int. Cl.: H04N 1/031, H04N 1/04, A61L 2/10

(54) **IMAGE SENSING APPARATUS, AND IMAGE SCANNING DEVICE AND SCANNING METHOD THEREOF**

(30) Priority: 25.06.2021 CN 202110714981
(71) Applicant: Weihai Hualing Opto-Electronics Co., Ltd., Weihai, Shandong 264209 (CN)
(72) Inventor: QI, Wuchang, Weihai, Shandong 264209 (CN); DENG, Juan, Weihai, Shandong 264209 (CN); QIU, Xiao, Weihai, Shandong 264209 (CN); WANG, Min, Weihai, Shandong 264209 (CN); SUN, Xiaofeng, Weihai, Shandong 264209 (CN)
(74) Representative: Grassi, Stefano
(86) International application number: PCT/CN2022/096967
(87) International publication number: WO 2022/267862

(57) **Abstract**

An image sensing apparatus, an image scanning device and a scanning method of the image scanning device are provided. The image sensing apparatus includes an image sensing assembly configured to sterilize and scan an object to be scanned, the image sensing assembly including a frame having an accommodating cavity and an opening communicating with the accommodating cavity; a contact image sensor, configured to read image information of the object to be scanned, the contact image sensor including detection light sources configured to emit detection beams; and a sterilization module, the sterilization module and the contact image sensor being sequentially arranged in the accommodating cavity in a first direction, and the sterilization module including an ultraviolet light source capable of emitting ultraviolet light to sterilize the object to be scanned. The technical solution of the invention solves the problem that the image sensing apparatus in the related art is difficult to fully sterilize the object to be scanned.

## Description

### Cross-Reference to Related Application

The invention claims priority to Patent Application No. 202110714981.6, filed to the China National Intellectual Property Administration on June 25, 2021 and entitled "Image Sensing Apparatus, Image Scanning Device and Scanning Method of Image Scanning Device".

### Technical Field

The invention relates to the technical field of image scanning, in particular to an image sensing apparatus, an image scanning device and a scanning method of the image scanning device.

### Background

Contact image sensors for scanning objects to be scanned are widely used in image reading devices such as scanners, multifunctional all-in-one machines, banknote reading authenticity identification instruments and ticket scanners. The objects to be scanned such as banknotes, tickets and documents may carry bacteria and viruses, and the process of scanning these objects to be scanned on a scanning device is likely to cause a spread of bacteria and viruses.

In order to avoid the spread of viruses and bacteria carried by the objects to be scanned, the image reading devices in a related art generally scan and sterilize the objects to be scanned at the same time using intermittent detection light emitted by a detection light source during the scanning process, which has some sterilization effect. However, an illumination time of the detection light source for scanning is relatively short, so that it is difficult to achieve the purpose of full sterilization.

### Summary

The invention mainly aims to provide an image sensing apparatus, an image scanning device and a scanning method of the image scanning device, so as to solve the problem that the image sensing apparatus in the related art is difficult to fully sterilize an object to be scanned.

According to an aspect of the invention, some embodiments of the invention provide an image sensing apparatus, which includes: an image sensing assembly configured to sterilize and scan an object to be scanned, the image sensing assembly includes a frame having an accommodating cavity and an opening communicating with the accommodating cavity; a contact image sensor, configured to read image information of the object to be scanned, the contact image sensor includes detection light sources configured to emit detection beams; and a sterilization module, the sterilization module and the contact image sensor being sequentially arranged in the accommodating cavity in a first direction, and the sterilization module including an ultraviolet light source capable of emitting ultraviolet light to sterilize the object to be scanned.

Further, the image sensing assembly further includes a baffle plate located in the accommodating cavity, the baffle plate is arranged between the sterilization module and the contact image sensor to separate the accommodating cavity into a first cavity and a second cavity, the sterilization module is located in the first cavity, and the contact image sensor is located in the second cavity.

Further, the image sensing assembly further includes a polarizer located on a side of the ultraviolet light source toward the opening, so that the ultraviolet light emitted by the ultraviolet light source is emitted at a preset angle in a direction far away from the detection light sources.

Further, the image sensing apparatus further includes: a position detection part, configured to detect a position of the object to be scanned, at least one side of the frame in the first direction being provided with the position detection part; and a controller, the ultraviolet light source, the contact image sensor and the position detection part being connected with the controller, and the controller controlling the opening and closing of the ultraviolet light source and the contact image sensor according to a detection signal transmitted by the position detection part.

Further, the image sensing assembly further includes a light-transmitting plate located at the opening, the light-transmitting plate being connected with the frame.

Further, the image sensing apparatus includes two image sensing assemblies arranged oppositely. The two image sensing assemblies are respectively located on opposite sides of the object to be scanned, so as to simultaneously sterilize and scan both sides of the object to be scanned.

Further, the contact image sensor further includes: a lens, configured to converge the detection beams reflected by the object to be scanned; a substrate, connected with the frame; and a photosensitive member, arranged on the substrate. The lens, the photosensitive member and the substrate are sequentially arranged in the accommodating cavity in a second direction perpendicular to the first direction, and the detection beams emitted by the detection light sources are reflected by the object to be scanned and are emitted into the photosensitive member after being converged by the lens.

Further, opposite sides of the lens in the first direction are provided with the detection light sources.

According to another aspect of the invention, some embodiments of the invention provide an image scanning device, which includes the above image sensing apparatus and a conveying apparatus configured to convey an object to be scanned.

Further, the conveying device includes: a first conveying part, including two first rollers arranged at an interval, the interval between the two first rollers forming a first conveying channel for conveying the object to be scanned, and rotation directions of the two first rollers being opposite; and a second conveying part, including two second rollers arranged at an interval, the interval between the two second rollers forming a second conveying channel corresponding to the first conveying channel for conveying the object to be scanned, and rotation directions of the two second rollers being opposite, where the first roller and the second roller located on the same side of the object to be scanned have the same rotation direction.

According to another aspect of the invention, some embodiments of the invention provide a scanning method of an image scanning device. The scanning method of the image scanning device sterilizes and scans an object to be scanned using the above image scanning device. The scanning method of the image scanning device includes: a sterilization step of sterilizing the object to be scanned through the sterilization module; and an acquisition step of acquiring image information of the object to be scanned through the contact image sensor.

Further, before the sterilization step and/or after the acquisition step, the scanning method of the image scanning device further includes a detection step of detecting a position of the object to be scanned using a position detection part.

By applying the technical solution of the invention, the side of the contact image sensor is additionally provided with the sterilization module, and the sterilization module includes the ultraviolet light source capable of emitting the ultraviolet light, so that before the contact image sensor scans the object to be scanned, or after the contact image sensor scans the object to be scanned, the object to be scanned is directly and continuously sterilized using the ultraviolet light emitted by the ultraviolet light source, and the object to be scanned is more fully sterilized.

### Brief Description of the Drawings

The drawings of the specification, which constitute a part of the invention, are intended to provide a further understanding of the invention, and the exemplary embodiments of the invention and the description thereof are intended to explain the invention and do not constitute an undue limitation on the invention. In the drawings:
Fig. 1 shows a schematic structural diagram of Embodiment 1 of an image scanning device according to the invention.
Fig. 2 shows a schematic structural diagram of Embodiment 2 of the image scanning device according to the invention.
Fig. 3 shows a schematic structural diagram of Embodiment 3 of the image scanning device according to the invention.
Fig. 4 shows a flowchart of an image scanning method according to an embodiment of the invention.
Fig. 5 shows a working time sequence diagram of an image scanning device when an object to be scanned enters a sterilization area according to an embodiment of the invention.
Fig. 6 shows a working time sequence diagram of an image scanning device when an object to be scanned leaves a scanning area according to an embodiment of the invention.

Herein, the above drawings include the following reference signs.
11. Contact image sensor; 111. Detection light source; 112. Lens; 113. Photosensitive member; 12. Sterilization module; 13. Baffle plate; 14. Frame; 15. Light-transmitting plate; 16. Position detection part; 17. First roller; 18. Second roller; 19. Object to be scanned; 20. Ultraviolet light source; 22. Polarizer.

### Detailed Description of the Embodiments

It is to be noted that the embodiments in the invention and features in the embodiments may be combined with each other without conflict. The invention is described below with reference to the drawings and in conjunction with the embodiments in detail.

It is to be noted that, compared with an image sensing apparatus in a related art for intermittently sterilizing an object to be scanned using a detection beam during scanning, the image sensing apparatus in the embodiments of the invention is additionally provided with a sterilization module, and ultraviolet light emitted by an ultraviolet light source of the sterilization module may directly and continuously irradiate the object to be scanned for sterilization, so that the object to be scanned is fully sterilized.

It is to be noted that, in the embodiments of the invention, detection light sources for scanning of a contact image sensor in the related art generally adopt Ultraviolet Radiation A (UVA) ultraviolet light, which is low in intensity and relatively low in sterilization effect.

It is to be noted that, in the embodiments of the invention, taking the contact image sensor with a resolution of 300 Dots Per Inch (DPI) as an example, a step distance of each line is 0.08 mm, and the length of a banknote is about 156 mm. In the process of sterilizing the object to be scanned using ultraviolet light in an Ultraviolet Radiation C (UVC) band, due to the fact that the ultraviolet light irradiates all corners of a surface of the object to be scanned, an area covered by the ultraviolet light is about 0.08×156 = 12.5 mm² = 0.125 cm² for each step distance scanned by the contact image sensor.

In this way, according to the power of a Light Emitting Diode (LED) array of the UVC ultraviolet light, the area covered by the ultraviolet light and the time required for scanning each step distance, the irradiation dose of the ultraviolet light of the image sensing apparatus in the embodiments of the invention is calculated. According to the relevant information consulted by the inventor, when killing general vegetative forms of bacteria, the irradiation dose of the ultraviolet light should reach 10000 uws/cm², and when killing bacterial spores, the irradiation dose of the ultraviolet light should reach 100000 uws/cm². The resistance of viruses to the ultraviolet light is between the vegetative forms of bacteria and the spores, the resistance of fungal spores is stronger than that of the bacterial spores, and the irradiation dose of the ultraviolet light should reach 600000uws/cm². Therefore, by comparing the irradiation dose of the ultraviolet light of the image sensing apparatus in the embodiments of the invention with the above parameters, the sterilization effect achieved by the embodiments of the invention may be obtained. Furthermore, according to the shortest time required for scanning each step distance by the contact image sensor, the speed of movement of the object to be scanned in a first direction, i.e., the time for the object to be scanned to pass through the image sensing apparatus, is determined. Further, the greater the power of the LED array of the UVC ultraviolet light, the better the sterilization effect, so that the speed of movement of the object to be scanned in the first direction is also improved.

It is to be noted that, in the embodiments of the invention, the UVC ultraviolet light refers to short-wave ultraviolet light with a wavelength ranging from 200 nm to 280 nm in the ultraviolet light.

It is to be noted that, in the embodiments of the invention, the sterilization module 12 and the contact image sensor 11 are controlled separately, work independently, and do not affect each other. In the occasions where image scanning is not required, only the object to be scanned is sterilized, and in the occasions where sterilization is not required, only the scanning operation is performed, so that the flexibility and convenience for the use of the image sensing apparatus are improved.

### Embodiment 1

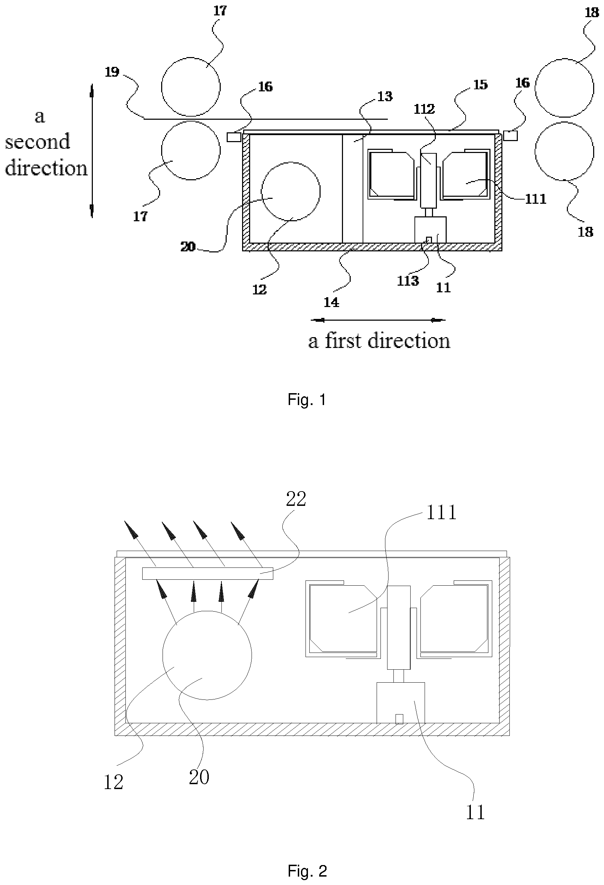

As shown in Fig. 1, Embodiment 1 of the invention provides an image sensing apparatus. The image sensing apparatus includes an image sensing assembly configured to sterilize and scan an object to be scanned 19. The image sensing assembly includes a frame 14, a contact image sensor 11 and a sterilization module 12. The frame 14 has an accommodating cavity and an opening communicating with the accommodating cavity. The contact image sensor 11 is configured to read image information of the object to be scanned 19, and the contact image sensor 11 includes at least one detection light source 111 configured to emit detection beams. The sterilization module 12 and the contact image sensor 11 are sequentially arranged in the accommodating cavity in a first direction, and the sterilization module 12 includes an ultraviolet light source 20 capable of emitting ultraviolet light to sterilize the object to be scanned 19.

In the above technical solution, the side of the contact image sensor 11 is additionally provided with the sterilization module 12, and the sterilization module 12 includes the ultraviolet light source 20 capable of emitting the ultraviolet light, so that before the contact image sensor 11 scans the object to be scanned 19, or after the contact image sensor 11 scans the object to be scanned 19, the object to be scanned 19 is directly and continuously sterilized using the ultraviolet light emitted by the ultraviolet light source 20, and the object to be scanned 19 is more fully sterilized to improve the sterilization effect.

In Embodiment 1 of the invention, the object to be scanned moves from left to right in the first direction in Fig. 1, and the image sensing apparatus first sterilizes the object to be scanned 19 using the ultraviolet light source 20, and then scans the object to be scanned 19 using the contact image sensor 11. Of course, in alternative embodiments not shown in the figures, the object to be scanned moves from right to left in the first direction in Fig. 1, so that the object to be scanned 19 is scanned using the contact image sensor 11, and then the object to be scanned 19 is sterilized by the ultraviolet light source 20.

In Embodiment 1 of the invention, the ultraviolet light source irradiates the object to be scanned using UVC light to achieve the sterilization function.

Specifically, in Embodiment 1 of the invention, the ultraviolet light source adopts full-time illumination, rather than pulse illumination in the process of scanning each line of the object to be scanned by the contact image sensor. Therefore, Embodiment 1 greatly improves the irradiation time of the ultraviolet light source, achieves the full sterilization effect, and maximizes the scanning speed on the basis of ensuring the sterilization effect.

As shown in Fig. 1, in Embodiment 1 of the invention, the image sensing assembly further includes a baffle plate 13 located in the accommodating cavity. The baffle plate 13 is arranged between the sterilization module 12 and the contact image sensor 11, so as to separate the accommodating cavity into a first cavity and a second cavity, wherein the sterilization module 12 is located in the first cavity, and the contact image sensor 11 is located in the second cavity.

Through the above arrangement, the baffle plate 13 arranged in the accommodating cavity may separate the ultraviolet light emitted by the sterilization module 12 from the detection beam emitted by the contact image sensor 11, so as to avoid the ultraviolet light interfering with the detection beam scanning the object to be scanned, and avoid the degradation of the quality of images output by the contact image sensor.

As shown in Fig. 1, in Embodiment 1 of the invention, the image sensing apparatus further includes a position detection part 16 and a controller. The position detection part 16 is configured to detect a position of the object to be scanned 19, at least one side of the frame 14 in the first direction is provided with the position detection part 16. The ultraviolet light source 20, the contact image sensor 11 and the position detection part 16 are connected with the controller, and the controller controls the opening and closing of the ultraviolet light source 20 and the contact image sensor 11 according to a detection signal transmitted by the position detection part 16.

Through the above arrangement, when the object to be scanned 19 moves in the first direction until the position detection part 16 detects that the object to be scanned 19 enters a sterilization area of the sterilization module 12, the position detection part 16 sends the detection signal to the controller, and the controller controls the opening and closing of the ultraviolet light source 20 and the contact image sensor 11, so that the ultraviolet light source 20 and the contact image sensor 11 sterilize and scan the object to be scanned 19 successively.

Specifically, as shown in Fig. 1, in Embodiment 1 of the invention, the number of position detection parts 16 is two, and the two position detection parts 16 are respectively located on the opposite sides of the frame 14 in the first direction. When the position detection part 16 on the left side in Fig. 1 detects that the object to be scanned enters the sterilization area (the range that the sterilization module 12 may irradiate), the ultraviolet light source 20 of the sterilization module 12 is turned on to sterilize the object to be scanned 19. When the object to be scanned 19 continues to move to a reading area (scanning area) of the contact image sensor 11, the contact image sensor 11 is turned on to scan the object to be scanned 19 and read the images. Then, as the object to be scanned 19 further moves in the first direction, when the position detection part 16 on the right side in Fig. 1 detects that the object to be scanned 19 leaves the sterilization area, the ultraviolet light source 20 of the sterilization module 12 is turned off, that is, the sterilization of the object to be scanned 19 is completed. When the object to be scanned 19 continues to move in the first direction until it leaves the reading area of the contact image sensor 11, the contact image sensor 11 is turned off to complete the image reading of the object to be scanned 19. In this way, on the one hand, sufficient sterilization time is ensured to achieve the full sterilization of the object to be scanned 19, on the other hand, the ultraviolet light source is effectively utilized to reduce the ineffective luminescence time, so as to reduce the temperature of the ultraviolet light source 20, thereby prolonging a service life of the ultraviolet light source 20.

As shown in Fig. 1, in Embodiment 1 of the invention, the image sensing assembly further includes a light-transmitting plate 15 located at the opening. The light-transmitting plate 15 is connected with the frame 14.

Through the above arrangement, the light-transmitting plate 15 and the frame 14 are able to seal the sterilization module 12 and the contact image sensor 11 in the accommodating cavity, so as to avoid dust entering the accommodating cavity, thereby avoiding an external environment affecting the work of the sterilization module 12 and the contact image sensor 11.

As shown in Fig. 1, in Embodiment 1 of the invention, the contact image sensor 11 further includes a lens 112, a substrate and a photosensitive member 113. The lens 112 is configured to converge the detection beams reflected by the object to be scanned 19. The substrate is connected with the frame 14. The photosensitive member 113 is arranged on the substrate. The lens 112, the photosensitive member 113 and the substrate are sequentially arranged in the accommodating cavity in a second direction perpendicular to the first direction, and the detection beams emitted by the detection light sources 111 are reflected by the object to be scanned 19 and are emitted into the photosensitive member 113 after being converged by the lens 112.

In the above technical solution, the detection beams emitted by the detection light sources 111 irradiate the object to be scanned 19 and are emitted into the lens 112 after being reflected by the object to be scanned 19. The lens 112 converges the reflected light and then the reflected light is irradiated onto the photosensitive member 113, and then the photosensitive member 113 converts a received light signal into an electrical signal, so that the contact image sensor 11 achieves the image reading function.

In Embodiment 1 of the invention, the detection beams emitted by the detection light sources 111 may be visible light (Red Green Blue (RGB)) or invisible light (Infrared (IR)) or ultraviolet light (UVA) or a combination thereof.

As shown in Fig. 1, in Embodiment 1 of the invention, the opposite sides of the lens 112 in the first direction are provided with the detection light sources 111.

Through the above arrangement, the detection light beams emitted by the detection light sources 111 on both sides may scan the object to be scanned 19, so that the scanning range is more comprehensive, and the intensity of the detection light beams is improved, so as to improve the picture quality of the scanned image.

As shown in Fig. 1, Embodiment 1 of the invention further provides an image sensing device. The image scanning device includes the above image sensing apparatus and a conveying apparatus configured to convey the object to be scanned 19.

Through the above arrangement, the object to be scanned 19 is conveyed using the conveying device, so that the object to be scanned 19 moves in the first direction, and the object to be scanned 19 passes through a sterilization area and a reading area.

As shown in Fig. 1, in Embodiment 1 of the invention, the conveying apparatus includes a first conveying part and a second conveying part. The first conveying part includes two first rollers 17 arranged at an interval. The interval between the two first rollers 17 forms a first conveying channel for conveying the object to be scanned 19, and rotation directions of the two first rollers 17 are opposite. The second conveying part includes two second rollers 18 arranged at an interval. The interval between the two second rollers 18 forms a second conveying channel for conveying the object to be scanned 19, wherein the second conveying channel corresponds to the first conveying channel, and rotation directions of the two second rollers 18 are opposite. The first roller 17 and the second roller 18 located on the same side of the object to be scanned 19 have the same rotation direction.

In the above technical solution, the two first rollers 17 of the first conveying part enable the object to be scanned 19 to move to the sterilization area along the first direction, so that the sterilization module 12 sterilizes the object to be scanned 19, and then the two first rollers 17 continue to convey the object to be scanned 19 to enter a scanning area, so that the contact image sensor 11 scans the object to be scanned 19. The two second rollers 18 of the second conveying part enable the object to be scanned 19 to move along the first direction and leave the sterilization area and the scanning area. By arranging the first conveying part and the second conveying part, the object to be scanned 19 moves more smoothly in the first direction.

Of course, in alternative embodiments not shown in the figures, the conveying apparatus includes the first conveying part and the second conveying part. The first conveying part includes one first roller 17 located on the side of the object to be scanned 19. The second conveying part includes one second roller 18. The first roller 17 and the second roller 18 are located on the same side of the object to be scanned 19, the first roller 17 and the second roller 18 have the same rotation direction, and the first roller 17 and the second roller 18 are located on the opposite sides of an image sensing assembly in a movement direction of the object to be scanned 19. In this way, the purpose of enabling the object to be scanned 19 to move in the first direction is also achieved.

### Embodiment 2

As shown in Fig. 2, Embodiment 2 of the invention differs from Embodiment 1 in that in Embodiment 2, a baffle plate 13 is not provided, but a polarizer 22 is provided. Specifically, in order to avoid ultraviolet light emitted by an ultraviolet light source 20 interfering with a detection light beam emitted by a contact image sensor, the image sensing assembly is provided with the polarizer 22 located on the side of the ultraviolet light source 20 toward an opening, so that the ultraviolet light emitted by the ultraviolet light source 20 is emitted at a preset angle in a direction far away from detection light sources 111.

Through the above arrangement, part of the ultraviolet light emitted by the ultraviolet light source 20 passes through the polarizer 22 and vibrates in a direction far away from the detection light sources 111, and then is emitted into an object to be scanned 19. Another part of the ultraviolet light emitted by the ultraviolet light source 20 is absorbed or reflected by the polarizer 22, so as to not only avoid a problem that the ultraviolet light interferes with the detection beam of the contact image sensor 11, but also sterilize the object to be scanned 19.

In Embodiment 2 of the invention, the polarizer 22 is located above the ultraviolet light source 20, so that all ultraviolet light emitted by the ultraviolet light source 20 first passes through the polarizer 22 and then is emitted to the object to be scanned 19.

In Embodiment 2 of the invention, the polarizer 22 is a polaroid, and the main function of the polaroid is to make the natural light become polarized light through the polaroid, that is, the polaroid allows the light that vibrates in a specific direction to pass through, so that the type of the polaroid is selected according to a required emergence angle of the ultraviolet light.

Other structures of the image scanning device in Embodiment 2 of the invention are the same as those in Embodiment 1, which will not be elaborated herein.

Of course, in some embodiments not shown in the figures, the polarizer 22 (that is, the image sensing apparatus both includes the polarizer 22 and the baffle plate 13) may also be provided on the basis of the structure of Embodiment 1, and the polarizer 22 is located on the side of the ultraviolet light source 20 toward the opening, so that the polarizer 22 allows the ultraviolet light that vibrates in the direction far away from the detection light sources 111 to pass through, and the baffle plate 13 separates the ultraviolet light emitted by the sterilization module 12 from the detection light beam emitted by the contact image sensor 11, so as to further avoid the ultraviolet light interfering with the detection light beam scanning the object to be scanned.

### Embodiment 3

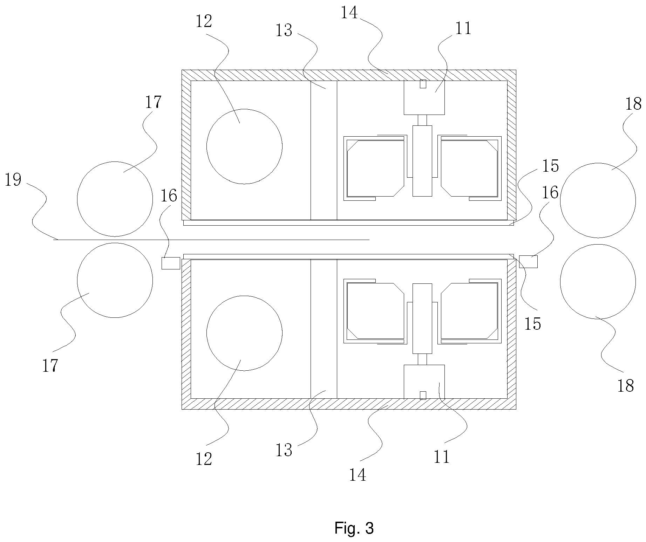

As shown in Fig. 3, Embodiment 3 of the invention differs from Embodiment 1 in that an image sensing apparatus of an image scanning device includes two image sensing assemblies arranged oppositely. The two image sensing assemblies are respectively located on the two opposite sides of the object to be scanned 19 to simultaneously sterilize and scan both sides of the object to be scanned 19.

Through the above arrangement, a channel is formed between the two image sensing assemblies. When the object to be scanned 19 passes through the channel in a first direction under the drive of a conveying device, the image sensing assemblies on both sides simultaneously scan and sterilize both sides of the object to be scanned 19, thereby improving the work efficiency of the image scanning device.

Other structures of the image scanning device in Embodiment 3 of the invention are the same as those of Embodiment 1, which will not be elaborated herein.

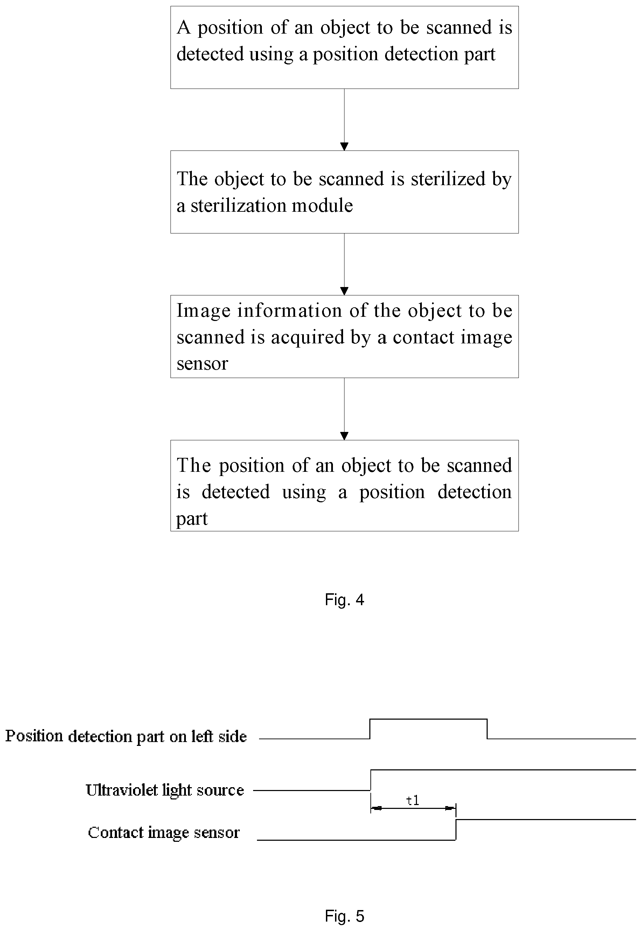

As shown in Fig. 4, the embodiments of the invention further provide a scanning method of an image scanning device. The scanning method of the image scanning device sterilizes and scans an object to be scanned using the image scanning device. The scanning method of the image scanning device includes: a sterilization step of sterilizing the object to be scanned 19 by the sterilization module 12; and an acquisition step of acquiring image information of the object to be scanned 19 by the contact image sensor 11.

In the above technical solution, the object to be scanned 19 is first sterilized using the sterilization module 12, and then the image information of the object to be scanned 19 is acquired using the contact image sensor 11. In this way, the object to be scanned is sterilized while the information of the object to be scanned is read using the contact image sensor 11.

As shown in Fig. 4, in the embodiments of the invention, before the sterilization step and/or after the acquisition step, the scanning method of the image scanning device further includes a detection step of detecting a position of the object to be scanned 19 by a position detection part 16.

Through the above arrangement, the position of the object to be scanned 19 is detected using the position detection part 16, when the object to be scanned 19 moves to a sterilization area of the sterilization module 12 in a first direction, or when the object to be scanned 19 leaves a scanning area of the contact image sensor 11 in the first direction, the position detection part 16 sends a detection signal to a controller, and the controller controls the opening and closing of the ultraviolet light source 20 and the contact image sensor 11, so that the ultraviolet light source 20 and the contact image sensor 11 sterilize and scan the object to be scanned 19 successively.

As shown in Fig. 1 and Fig. 5, in the embodiments of the invention, when a front edge of the object to be scanned 19 passes above the position detection part 16 on the left side in Fig. 1, that is, the object to be scanned 19 enters the sterilization area, an output signal of the position detection part 16 changes from a low level to a high level, the ultraviolet light source 20 of the sterilization module 12 changes from a low level to a high level, and the ultraviolet light source 20 is turned on and enters a sterilization mode. At a first preset time t1 after the front edge of the object to be scanned 19 passes through the detection part 16 on the left side in Fig. 1, the object to be scanned 19 enters a scanning area of the contact image sensor 11, the signal of the contact image sensor 11 changes to the high level, and the contact image sensor 11 enables the image reading function and scans the object to be scanned.

It is to be noted that, in the embodiments of the invention, the first preset time t1 is a ratio of a length of the sterilization area in the first direction to the speed of movement of the object to be scanned in the first direction.

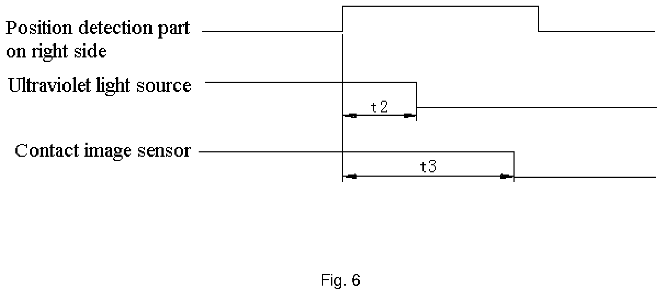

As shown in Fig. 1 and Fig. 6, in the embodiments of the invention, when the front edge of the object to be scanned 19 passes above the position detection part 16 on the right side in Fig. 1, that is, the object to be scanned 19 starts to leave the scanning area, the output signal of the position detection part 16 changes from the low level to the high level, and the signal of the ultraviolet light source 20 of the sterilization module 12 changes to the low level after a second preset time t2. At this time, after the object to be scanned 19 leaves the irradiation range (i.e., the sterilization area) of the ultraviolet light source 20, the ultraviolet light source 20 is turned off, the sterilization module 12 stops sterilization, and the signal of the contact image sensor 11 changes to the low level after a third preset time t3. At this time, when the object to be scanned 19 leaves the reading range (i.e., the scanning area) of the contact image sensor 11, the contact image sensor 11 stops the image reading function and completes the image reading of the object to be scanned.

It is to be noted that, in the embodiments of the invention, the second preset time t2 is obtained by dividing a difference between the length of the object to be scanned in the first direction and the length of the scanning area in the first direction by the speed of movement of the object to be scanned in the first direction. The third preset time t3 is a ratio of the length of the object to be scanned 19 in the first direction to the speed of movement of the object to be scanned in the first direction.

From the above description, it can be seen that the above embodiments of the invention achieve the following technical effects: the side of the contact image sensor is additionally provided with the sterilization module, and the sterilization module includes the ultraviolet light source capable of emitting the ultraviolet light, so that before the contact image sensor scans the object to be scanned, the object to be scanned is directly and continuously sterilized using the ultraviolet light emitted by the ultraviolet light source, and the object to be scanned is more fully sterilized.

The above is only some embodiments of the invention, and is not intended to limit the invention, and for those of ordinary skill in the art, various modifications and changes may be made to the invention. Any modifications, equivalent substitutions, improvements, etc. within the spirit and scope of the invention shall be included in the scope of protection of the invention.

## Claims

1. An image sensing apparatus, comprising an image sensing assembly configured to sterilize and scan an object to be scanned (19), the image sensing assembly comprising:
a frame (14), having an accommodating cavity and an opening communicating with the accommodating cavity;
a contact image sensor (11), configured to read image information of the object to be scanned (19), the contact image sensor (11) comprising detection light sources (111) configured to emit detection beams; and
a sterilization module (12), the sterilization module (12) and the contact image sensor (11) being sequentially arranged in the accommodating cavity in a first direction, and the sterilization module (12) comprising an ultraviolet light source (20) capable of emitting ultraviolet light to sterilize the object to be scanned (19).

2. The image sensing apparatus according to claim 1, wherein the image sensing assembly further comprises a baffle plate (13) located in the accommodating cavity, wherein the baffle plate (13) is arranged between the sterilization module (12) and the contact image sensor (11) to separate the accommodating cavity into a first cavity and a second cavity, the sterilization module (12) is located in the first cavity, and the contact image sensor (11) is located in the second cavity.

3. The image sensing apparatus according to claim 1, wherein the image sensing assembly further comprises a polarizer (22) located on a side of the ultraviolet light source (20) toward the opening, so that the ultraviolet light emitted by the ultraviolet light source (20) is emitted at a preset angle in a direction far away from the detection light sources (111).

4. The image sensing apparatus according to any one of claims 1 to 3, further comprising:
a position detection part (16), configured to detect a position of the object to be scanned (19), at least one side of the frame (14) in the first direction being provided with the position detection part (16); and
a controller, the ultraviolet light source (20), the contact image sensor (11) and the position detection part (16) being connected with the controller, and the controller controlling the opening and closing of the ultraviolet light source (20) and the contact image sensor (11) according to a detection signal transmitted by the position detection part (16).

5. The image sensing apparatus according to any one of claims 1 to 3, wherein the image sensing assembly further comprises a light-transmitting plate (15) located at the opening, the light-transmitting plate (15) being connected with the frame (14).

6. The image sensing apparatus according to any one of claims 1 to 3, comprising two image sensing assemblies arranged oppositely, the two image sensing assemblies being respectively located on opposite sides of the object to be scanned (19), so as to simultaneously sterilize and scan both sides of the object to be scanned (19).

7. The image sensing apparatus according to any one of claims 1 to 3, wherein the contact image sensor (11) further comprises:
a lens (112), configured to converge the detection beams reflected by the object to be scanned (19);
a substrate, connected with the frame (14); and
a photosensitive member (113), arranged on the substrate, wherein the lens (112), the photosensitive member (113) and the substrate are sequentially arranged in the accommodating cavity in a second direction perpendicular to the first direction, and the detection beams emitted by the detection light sources (111) are reflected by the object to be scanned (19) and are emitted into the photosensitive member (113) after being converged by the lens (112).

8. The image sensing apparatus according to claim 7, wherein opposite sides of the lens (112) in the first direction are provided with the detection light sources (111).

9. An image scanning device, comprising the image sensing apparatus according to any one of claims 1 to 8 and a conveying apparatus configured to convey the object to be scanned (19).

10. The image scanning device according to claim 9, wherein the conveying apparatus comprises:
a first conveying part, comprising two first rollers (17) arranged at an interval, the interval between the two first rollers (17) forming a first conveying channel for conveying the object to be scanned (19), and rotation directions of the two first rollers (17) being opposite; and
a second conveying part, comprising two second rollers (18) arranged at an interval, the interval between the two second rollers (18) forming a second conveying channel corresponding to the first conveying channel for conveying the object to be scanned (19), and rotation directions of the two second rollers (18) being opposite, wherein the first roller (17) and the second roller (18) located on a same side of the object to be scanned (19) have the same rotation direction.

11. A scanning method of the image scanning device, wherein the scanning method of the image scanning device sterilizes and scans the object to be scanned using the image scanning device according to claim 9 or 10, and the scanning method of the image scanning device comprises:
a sterilization step of sterilizing the object to be scanned (19) through the sterilization module (12); and
an acquisition step of acquiring image information of the object to be scanned (19) through the contact image sensor (11).

12. The scanning method of the image scanning device according to claim 11, wherein before the sterilization step and/or after the acquisition step, the scanning method of the image scanning device further comprises a detection step of detecting a position of the object to be scanned (19) using a position detection part (16).
